# EUROPEAN PATENT SPECIFICATION

(11) **EP 1 579 745 B1**
(45) Date of publication and mention of the grant of the patent: **24.05.2006**
(21) Application number: 03807739.2
(22) Date of filing: 25.09.2003
(51) Int. Cl.: H05K 1/00

(54) **LAYERED STRUCTURE WITH ELECTRIC LEADS FOR A BODY WORN DEVICE**
SCHICHTSTRUKTUR MIT ELEKTRISCHEN LEITUNGEN FÜR EINE AM KÖRPER GETRAGENE EINRICHTUNG
STRUCTURE STRATIFIEE COMPRENANT DES FILS ELECTRIQUES DE SORTIE POUR DISPOSITIF PORTE SUR LE CORPS

(30) Priority: 08.10.2002 DK 200201512
(43) Date of publication of application: 28.09.2005
(73) Proprietor: OTICON A/S, 2765 Smoerum (DK)
(72) Inventor: MEHR, Ulrik, Strandvejen 58 DK-2900 Hellerup (DK); JOHANSEN, Jesper, B., Strandvejen 58 DK-2900 Hellerup (DK)
(86) International application number: PCT/DK2003/000630
(87) International publication number: WO 2004/034756

(56) References cited:
- US-A1- 2001 035 529
- US-B1- 6 396 000

## Description

### AREA OF THE INVENTION

The invention relates to a layered structure for a body warn device wherein electric signals are transported along metallic leads, which are adhered to a layer on or within the layered structure and where a first and a second lead for connecting a first and a second terminal of a component are provided. The circuit board may be a regular PCB or a flex print or other type of multilayer circuit. Such a layered structure is typically used in hearing aids, head sets and other small size body worn devices wherein electric signal processing takes place. Other types of body worn devices wherein the invention finds use is in pacemakers and insulin pumps. The device may be worn on any part of the human body.

### BACKGROUND OF THE INVENTION

In certain types of devices it is a problem, that the electric leads radiate magnetic and electric fields. These fields may disturb other parts of the circuitry or components mounted on or in relation to the circuit. Also the leads may pick up electric or magnetic fields from nearby parts of the circuit or from devices at or near the circuit board. It is known to arrange the first and second lead adjacent to one another, such that the currents which in this case will have opposite directions in the two lead produce electric and magnetic fields of opposite polarity, which to some degree will cancel out each other. This can be done by arranging the leads on each their side of a thin layer in the board and opposing each other. For certain applications this is however not enough to overcome the problems of electric and magnetic fields. One way of solving the problem is to use shields on one or both sides of the leads. This is however not practical in many cases, as more layers in the PCB will be required for this purpose resulting in both more expensive and more voluminous PCB's. Especially in hearing aids this is a big problem, as the extra demand for volume will result in bulkier and less attractive hearing aids. In hearing aids it is also known to use a pair of thin isolated wires for the leads in stead of providing leads on the circuit board, and in doing so the wires may be twisted around each other. This will reduce the radiation problems, as the twisted wires radiate much less energy, and have a strongly reduced ability to pick up radiation. The use of such wires is however very cumbersome, as they must be handled manually, which makes the production price of the apparatus rise. Also in manual operations of this nature some variations are bound to occur and this may result in some variation as to how well the units function.

It is the object of the invention to provide a layered structure with circuitry for a hearing aid wherein the above problems are solved.

### SUMMARY OF THE INVENTIO

According to the invention the problems associated with the prior art are solved by passing the two leads side by side and alternating on the two sides of a layer, and in such a manner that the first and second lead will cross one another at an angle but passing on each their side of the layer: In this manner the tow leads will pass along the layer in a double spiral, and at each twist of the spiral, the first and the second lead will pass across one another, but with the layer between them. In such a double spiral it is assured that the radiation from the leads becomes minimal, and that the ability to pick up electromagnetic radiation is minimized.

It is preferred to pass the leads in such a way that a maximum number of twists is achieved. In this way the electric and magnetic fields produced in each lead when audio frequency signals are passed through the leads will cancel each other most efficiently.

In an embodiment the leads from one through hole of the layer to the next are drawn in a straight line and the through holes for passing the leads through the layer are placed side by side with no more space there between than is necessary for isolation purposes.

In a preferred embodiment of the invention the leads are connected to an amplifier at one end and to a hearing aid receiver at the other end. When such a lead to the receiver is used, the radiation and pick up from the leads is so small that the telecoil may be placed at any desired place on the circuit board, without regards to the radiation from the leads connecting the receiver.

### BRIEF DESCRIPTION OF THE DRAWINGS

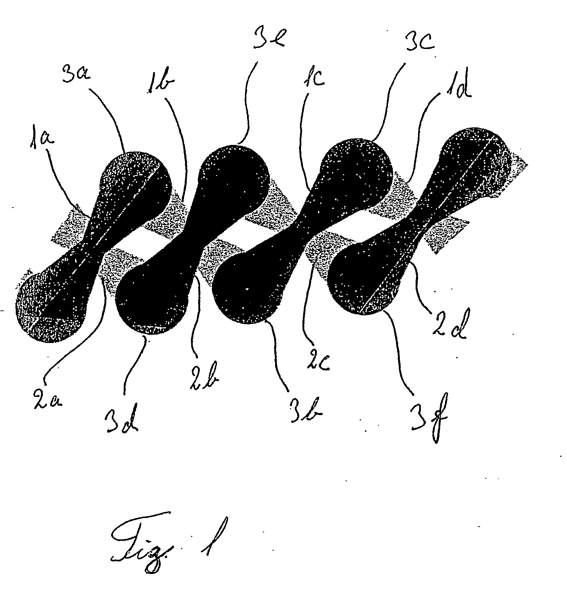

Fig. 1 shows an example of an embodiment of the invention on a PCB.

### DESCRIPTION OF A PREFERRED EMBODIMENT

Fig. 1 shows a front view of a part of a PCB layer. The leads marked in black 2a, 2b, 2c, 2d are on the front side of the layer, and the leads marked in grey 1a, 1b, 1c, 1d are on the back side. A lead 1a on the front side passes along a micro via 3a to the back side, on the back side in the path 1b, and back to the front side through the layer in micro via 3b, and on the front side lc. The other lead 2a passes on the back side to micro via 3d and to the front side, along the front side 2b to via 3e, on the back side 2c to another micro via 3f and back to the front side 2d.

Typically the lead 1a,1b,1c,1d connects a current consuming device like a hearing aid receiver with the amplifier, and the lead 2a,2b,2c and 2d is the return lead from the receiver to the amplifier. The pairs of leads 1a, 2a; 1b, 2b; 1c,2c all cross each other but always on each their side of the layer. The distance between the micro vias 3a and 3e in the length direction of the double lead is choses to be in the same order as the distance between the vias 3a and 3d in the sideways direction. The four vias 3a, 3e, 3d,3b then forms the corners of a square and the leads 1b and 2b connects the corners of this square.

The size of the square is chosen to be as small as possible, thereby placing the leads as close to one another as possible. In this way radiation and pick up is kept as small as possible.

It is however possible that other objects are placed on the print and thus this ideal placement of the leads cannot always be maintained.

In the above example the leads are printed on each their side of a print layer in a PCB, but they could also be embedded in the multi layer structure within or on an IC.

A layered structure as described above may be used in other kind of devices than hearing aids. This could be in any body worn electronic device, wherein problems of radiation of electromagnetic energy from electric leads is a problem.

## Claims

1. Layered structure with electric leads for a hearing aid , wherein electric signals are fed along metallic leads, which are adhered to a layer on or within the layered structure and where a first and a second lead for connecting a first and a second terminal of a component are provided and whereby the leads are connected to an amplifier at one end and to a hearing aid receiver at the other end and whereby the two leads are passed side by side and alternating on the two sides of the layer, and in such a manner that the first and second lead will cross one another at an angle but passing on each their side of the layer.

2. Layered structure as claimed in claim 1, where the leads pass in such a way that a maximum number of twists is achieved.

3. Layered structure as claimed in claim 1 whereby the leads from one through hole of the layer to the next are drawn in a straight line and the through holes for passing the leads through the layer are placed side by side with no more space there between than is necessary for isolation purposes.

## Patentansprüche

1. Geschichtete Struktur mit elektrischen Leitungen für eine Hörhilfe, wobei elektrische Signale längs metallischer Leitung geführt werden, welche an einer Schicht an oder innerhalb der geschichteten Struktur anhaften, wobei eine erste und eine zweite Leitung zur Verbindung eines ersten und eines zweiten Anschlusses einer Komponente vorgesehen sind, wobei die Leitungen mit einem Verstärker an einem Ende und mit einem Empfänger der Hörhilfe an dem anderen Ende verbunden sind und wobei die beiden Leitungen Seite an Seite geführt sind und auf den beiden Seiten der genannten Schicht wechseln, in solcher Weise, daß die erste Leitung und die zweite Leitung einander unter einem Winkel kreuzen jedoch jeweils auf ihrer Seite der Schicht verlaufen.

2. Geschichtete Struktur nach Anspruch 1, bei welcher die Leitungen in solcher Weise geführt sind, daß eine Maximalzahl von Verdrillungen erreicht wird.

3. Geschichtete Struktur nach Anspruch 1, bei welcher die Leitungen von einer Durchführungsöffnung der Schicht zur nächsten Durchführungsöffnung der Schicht in einer geraden Linie verlaufen und die Durchführungsöffnungen zum Hindurchführen der Leitungen durch die Schicht Seite an Seite angeordnet sind, wobei der Abstand dazwischen nicht größer ist als für Isolationzwecke notwendig ist.

## Revendications

1. Structure stratifiée de conducteurs électriques pour une aide auditive, dans laquelle des signaux électriques sont transmis le long de conducteurs métalliques qui sont collés à une couche située sur ou à l'intérieur de la structure stratifiée, où il est prévu des premier et second conducteurs destinés à se connecter à des première et seconde bornes d'un composant, où les conducteurs sont connectés à un amplificateur à une extrémité et à un récepteur d'aide auditive à l'autre extrémité, et où les deux conducteurs sont passés côte à côte et alternativement sur les deux côtés de la couche, et de telle manière que les premier et second conducteurs se croisent mutuellement selon un angle mais en passant chacun sur son côté de la couche.

2. Structure stratifiée selon la revendication 1, dans laquelle les conducteurs passent de telle manière qu'on obtienne un nombre maximum de tours de torsion.

3. Structure stratifiée selon la revendication 1, dans laquelle les conducteurs allant d'un trou débouchant de la couche au suivant sont tirés en une ligne droite et les trous débouchants servant à faire passer les conducteurs à travers la couche sont placés côte à côte avec pas plus d'espace entre eux qu'il n'en faut pour les besoins de l'isolation.
